# EUROPÄISCHE PATENTSCHRIFT

(11) **EP 0 714 026 B1**
(45) Veröffentlichungstag und Bekanntmachung des Hinweises auf die Patenterteilung: **02.01.2004**
(21) Anmeldenummer: 95116243.7
(22) Anmeldetag: 16.10.1995
(51) Int. Cl.: G01N 27/26, C12Q 1/70, G01N 1/30, G01N 33/52, G01N 33/569

(54) **Verfahren zur analytischen Trennung von Viren**
Method for the analytical separation of viruses
Méthode pour la séparation analytique de virus

(30) Priorität: 31.10.1994 DE 4438833
(43) Veröffentlichungstag der Anmeldung: 29.05.1996
(73) Patentinhaber: Dürr, Hansjoerg Dr., 51399 Burscheid (DE); Brüggemeier, Ulf Dr., 42799 Leichlingen (DE)
(72) Erfinder: Dürr, Hansjörg, D-51399 Burscheid (DE); Hehnen, Hans-Robert, Dr., D-53721 Siegburg (DE); Helbig, Lothar, D-51373 Leverkusen (DE); Correa, Roberto, D-51373 Leverkusen (DE); Brüggemeier, Ulf, Dr., D-42799 Leichlingen (DE)
(74) Vertreter: Gille Hrabal Struck Neidlein Prop Roos

(56) Entgegenhaltungen:
- WO-A-95/08640
- US-A- 4 181 589
- US-A- 5 045 172
- JOURNAL OF CHROMATOGRAPHY, Bd. 403, 21.August 1987, Seiten 47-61, XP000644988 S. HJERTEN ET AL.: "Carrier-free zone elctrophoresis, displacement electrophoresis and isoelectric focusing in a high-performance electrophoresis apparatus."

## Beschreibung

Die Erfindung geht aus von einem Verfahren zum zerstörungsfreien analytischen Nachweis und/oder zur Quantifizierung von Viren oder viralen Partikeln (Analyten) in einer organische oder anorganische Nebenkomponenten, insbesondere Proteinanteile und/oder Nucleotide und/oder andere Viren, enthaltenden flüssigen Probenmatrix.

Bekannt sind immunologische Verfahren die auf spezifischen Antigen-Antikörper-Wechselwirkungen beruhen. Die zahlreichen, angewandten Verfahren wie z. B. ELISA (Enzyme-Linked-Immuno-Sorbent-Assay), KBR (Komplement-Bindungs-Reaktion), SNT (Serum-Neutralisations-Test) und fluoreszensmarkierte Antiköper unterscheiden sich lediglich im Detektionsverfahren.

Problematisch an diesen Verfahren ist die hohe Spezifität der Nachweisreaktion auf den immunogenen Bereich des Virus. Auch kann nicht zwischen der Antigen-Aktivität des Viruspartikels und des immunreaktiven Proteins unterschieden werden. Umgekehrt können auch falsch positive Ergebnisse über Kreuzreaktivitäten auftreten.

Die spezifische virale Aktivität wird üblicherweise über zwei Effekte gemessen:
a) Es wird ein spezieller pathogener Effekts des Virus auf Zielzellen erfaßt.
   Beispielhaft kann hier die visuelle Kontrolle einer infizierten Zellpopulation auf Cytopathogenität, Hämagglutination oder Hämadsorption genannt werden.
b) Eine virusspezifische Enzymreaktion kann z. B. bei Retroviren über die Reverse-Transkriptase-Aktivität gemessen werden.

Problematisch ist hier die schlechte Quantifizierbarkeit und die Unsicherheit in der Relevanz der Testsysteme für die Übertragbarkeit auf den natürlichen Wirt.

Molekularbiologische Verfahren können wie folgt zusammengefaßt werden:
a) In der Sondendiagnostik werden virale Nucleotidsequenzen durch Hybridisierung mit eingebrachten spezifischen Gensonden erfaßt.
b) Virale Gene werden über ein spezifisches, durch Restriktionsenzyme erzeugtes Spaltmuster identifiziert.
c) Viren werden durch die Amplifikation spezifischer Genabschnitte identifiziert (PCR).
d) Virale Gene werden durch die Nukleotidsequenzierung identifiziert.

Diese Verfahren beinhalten den größten Informationsgehalt zur Virusidentität. Sie sind jedoch relativ aufwendig, arbeitsintensiv und für Quantifizierungen schlecht geeignet. Außerdem beschreiben diese Analysenverfahren den Genotyp und nicht den Phänotyp des Virus.

Das virusspezifische Proteinpattern kann durch die Auftrennung mit verschiedenen elektrophoretischen und chromatographischen Techniken erfaßt werden. Immunogene Proteine können zusätzlich durch immunologische Verfahren (Immunoblot) erkannt werden. Viren müssen vor der Spaltung sehr sauber sein (Reinigungsaufwand) um die Proteinbanden zuordnen zu können. Auch bereits zerstörte Viren können das richtige Pattern zeigen.

Die Kapillarelektrophorese (CE) ist eine relativ neue Trenntechnik, bei der die zu analysierende Probe in einer Kapillare im elektrischen Hochspannungsfeld aufgrund unterschiedlicher Migrationsgeschwindigkeiten getrennt werden. Die Detektion findet dabei direkt in der Kapillare unter Verwendung optischer Detektoren statt [1]. Die Anwendungsbreite der CE ist sehr groß und erstreckt sich über die Ionenanalytik, die Enantiomerentrennung, die Proteinanalytik, die Nukleotidanalytik bis zur Trennung von Partikeln [2] und Viren [10]. Bisher wurden damit hauptsächlich einzelne Moleküle oder einheitliche, mechanisch stabile Teilchen untersucht. In [3] wird z. B. die kapillarelektrophoretische Trennung von rekombinanten Lipoprotein-Partikeln beschrieben. In [10] wird die kappillarelektrophoretische Trennung eines Virus beschrieben.

Zur empfindlichen Fluoreszenzdetektion für DNA und RNA werden DNA/RNA bindende Anfärbereagenzien erfolgreich eingesetzt [4,5]. Diese Farbstoffe zeigen zum Teil unterschiedliche Selektivitäten für einzelsträngige oder doppelsträngige Nukleotide oder unterschiedliche Floureszenzmaxima für RNA und DNA. So zeigt zum Beispiel Acridinorange mit DNA ein Maximum in der Fluoreszenzemission bei 520 nm, mit RNA bei 650 nm. Die Fluoreszenzdetektion ist auch in der CE fast ausschließlich für doppelsträngige DNA beschrieben [6].

Zur Bestimmung von Bindungskonstanten mit der CE sind in der Literatur eine Vielzahl von Verfahren für Protein-Ligand-Wechselwirkungen beschrieben worden [7,8]. Die Verfahren beruhen entweder auf einer Verschiebung der Migrationszeit durch die Wechselwirkung, oder auf Änderungen in der Peakfläche eines Wechselwirkungspartners. Experimentell kann dies wie folgt realisiert werden:
1. Die Wechselwirkungspartner werden vor der CE-Trennung inkubiert und mit der CE die gebildeten Komplexe von den Einzelkomponenten getrennt.
2. Ein Wechselwirkungspartner wird im CE-Puffer vorgelegt und die Migrationszeitverschiebung des anderen Partners gemessen (Kapillaraffinitätselektrophorese).

Das erste Verfahren hat den Vorteil, substanzsparend und variabel in der Wahl der Wechselwirkungspartner zu sein und wurde daher primär untersucht.

Der Erfindung liegt die Aufgabe zugrunde, die analytische Untersuchung von Viren zu verbessern, sodaß eine schnelle und eindeutige Identifizierung und/oder Quantifizierung in einer organische oder anorganische Nebenkomponenten, insbes.

Proteinanteile und/oder Nucleotide und/oder andere Viren, enthaltenden flüssigen Probenmatrix ermöglicht wird.

Diese Aufgabe wird erfindungsgemäß dadurch gelöst, daß die Viren oder die viralen Partikel von den Proteinanteilen und/oder Nucleotiden in der Probenmatrix kapillarelektrophoretisch getrennt werden und dabei das zugehörige Elektropherogramm registriert wird und daß die den Analyten zugeordneten Fraktionen als Virus-Peaks im Elektropherogramm durch spektroskopische Auswertung an Hand charakteristischer Maxima identifiziert werden und die Viren oder die viralen Partikel mit einem RNA/DNA-bindenden, spektroskopisch identifizierbaren Farbstoff angefärbt werden.

Unter viralen Partikeln sind definitionsgemäß aktive und inaktivierte Viren, sowie chemisch, molekularbiologisch oder enzymatisch modifizierte Teilchen zu verstehen, die aufgrund ihrer Oberflächeneigenschaften, oder aufgrund ihres strukturellen Aufbaus als virusähnlich anzusehen sind. Die Viren (virale Partikel) können DNA oder RNA enthalten, doppelsträngig oder einzelsträngig, sie können umhüllt oder unbehüllt vorliegen. Die Trennung ist unabhängig von der Größe des Virus, auch Viroide oder Virionen können so charakterisiert werden.

Die Viren können dabei direkt aus einer beliebigen Probenmatrix, wie beispielsweise aus biologischem Material (Serum, Urin, Zellen, Plasma, Zellüberstand, Augenflüssigkeit, Speichel, usw.) oder aus nichtbiologischen Formulierungen (Wasser, Medikament, Bodenprobe, usw.) identifiziert werden.

Durch die Verwendung eines Fluoreszenzfarbstoffs, der fluoreszenzspektrometnsch detektiert wird, kann eine besonders hohe Empfindlichkeit erreicht werden.

Vorteilhaft wird der Probenmatrix zur Identifizierung eine Referenzprobe eines bekannten Virus oder eines bekannten viralen Partikels zugesetzt.

Eine weitere Verbesserung besteht darin, daß das Virus oder die viralen Partikel mit spezifischen Antikörpern inkubiert werden, die eine Verschiebung der elektrophoretischen Migrationszeit oder eine Änderung des Virus-Peaks hervorrufen.

Mit dem neuen Verfahren kann in vorteilhafter Weise die Herstellung der Viren bei der Vakzineproduktion meßtechnisch überwacht und gesteuert werden.

Eine weitere, sehr wichtige Anwendung besteht darin, daß Viren direkt in biologischen Materialien, insbesondere in Körperflüssigkeiten diagnostiziert werden können.

Mit dem erfindungsgemäßen Verfahren werden folgende Vorteile erzielt:

Gegenüber den vorhanden Analysenverfahren besticht die kapillarelektrophoretische Trennung der viralen Partikel durch die einfache Probenvorbereitung, die kurzen Analysenzeiten, die exakte Quantifizierbarkeit, die Automatisierbarkeit, die Standardisierbarkeit und die neue Qualität der Aussage, da erstmals eine Identifizierung und Quantifizierung intakter Viren ohne Einschränkung möglich ist.

Diese Vorteile wirken sich besonders stark aus:
a) bei der Prozeßkontrolle:
   Mit diesem Verfahren ist es aufgrund der schnellen und quantitativen Analyse erstmals möglich bei der Vakzineproduktion nicht nur den Prozeß zu kontrollieren, sondern auch zu steuern.
b) bei der Produktkontrolle:
   Damit kann erstmals mit einem einheitlichen Verfahren die Qualitätssicherung des Endprodukts von der Formulierung bis zur Überwachung der Lagerstabilität und der Transportbedingungen durchgeführt werden.
c) bei der Virusdiagnostik:
   Viren können mit diesem Verfahren erstmals direkt detektiert und identifiziert werden. Dies ist sowohl für die Produktüberwachung in der Virusproduktion, als auch für die Virusfreiheit in biologischen Materialien von großer Bedeutung.
d) bei der Therapieüberwachung:
   Beim Einsatz antiviraler Therapeutika kann der Therapieerfolg direkt verfolgt werden.
e) bei der Virusidentifizierung:

Durch die unterschiedlichen elektrophoretischen Eigenschaften der Viren und deren spezifische Detektierbarkeit in der Kapillare können bekannte Viren identifiziert und unbekannte festgestellt werden. Auch mehrere (unterschiedliche) Viren (Virusstämme) können so parallel erfasst werden.

Im Folgenden wird an Hand von Ausführungsbeispielen mit Meßkurven beschrieben, wie mit dem neuen Analysenverfahren Viren und virale Partikel kapillarelektrophoretisch getrennt direkt identifiziert und quantifiziert werden können.

Es zeigen:
- Abb. 1:: Kapillarelelektropherogramm einer inaktivierten MKS-Referenzprobe, Detektion bei 210 ± 8 nm (a) und 257 ± 8 nm (b). Das Virussignal kann durch das Anwachsen bei der Detektion bei 257 nm identifiziert werden
- Abb. 2:: DAD-Spektrum von inaktiviertem MKS-Virus aus Abbildung 1.
- Abb. 3:: Kapillarelelektropherogramm von inaktiviertem MKS-Konzentrat. Detektion bei 210 ± 8 nm (a) und 257 ± 8 nm (b). Das Virus ist durch den Pfeil gekennzeichnet.
- Abb. 4:: Vergleich der CE-Trennungen von inaktiven (a) und aktiven (b) MKS-Referenzproben. Detektion bei 257 nm.
- Abb. 5:: CE-Trennung von angefärbtem, inaktivem MKS-Virus aus Abbildung 1. Detektion bei 257 ± 8 nm. Das Signal bei 3.8 min konnte überschüssigem Ethidiumbromid aus der Inkubation zugeordnet werden. Das Virussignal wurde durch die Anfärbung nicht verschoben (Vergleich mit Abb. 1).
- Abb. 6:: DAD-Spektren von Ethidiumbromid (a) und dem angefärbtem, inaktivem MKS-Virus (b) aus Abbildung 5. Damit konnte die erfolgreiche Anfärbung des Virus bewiesen werden. Zusätzlich wurde damit das Virussignal spezifiziert.
- Abb. 7:: CE-Trennung von angefärbtem (YOYO), inaktivem MKS-Konzentrat aus Abbildung 3 mit LIF-Detektion. Aus der komplexen Mischung (Abb. 3) ist nur noch das Virus und deutlich empfindlicher detektierbar.
- Abb. 8:: Vergleich der CE-Trennungen von inaktivem Ort-Virus vor (a) und nach Inkubation mit einem Orf-spezifischen MAb (b). Das Virus wurde mit YOYO angefärbt und dem LIF detektiert.
- Abb. 9:: CE-Trennungen der Core des Orf-Virus nach Anfärbung mit YOYO und Detektion bei 257 nm (a) und LIF-Detektion (b). Das breite Signal bei 257 nm stammt von überschüßigem YOYO. Die unterschiedlichen Migrationszeiten kommen von unterschiedlichen Trennparametern.
- Abb. 10:: DAD-Spektrum der Core des Orf-Virus mit interkaliertem YOYO aus Abbildung 9. Durch Interkalation wurde ein Absorptionmaximum im Anregungsbereich des Argon-Lasers des LIF-Detektors (495 nm) erzeugt.
- Abb. 11:: Vergleich der relativen Migrationszeiten von MKS-O₁ₖ(a) und MKS-O₁ₘ(b) ohne MAb (dunkle Balken) und nach Inkubation mit unterschiedlichen Anti-MKS-O₁-MAb (helle Balken).

### Allgemeine Verfahrensbeschreibung

Die zu analysierende Probe wird in flüssiger Form (Lösung, Suspension) in eine Kapillare eingebracht, die vorher mit dem Trennpuffer gespült wurde oder mit einem Puffersystem gefüllt wurde. An den beiden Kapillarenden wird über direkten Kontakt, oder über einen leitenden Puffer der Stromtransport gewährleistet und durch das Anlegen einer Hochspannung in der Kapillare ein elektrisches Feld erzeugt. Die Viren werden im elektrischen Feld getrennt. Die direkte Identifizierung der Viren in der Kapillare erfolgt durch Detektion der UV-Absorption bei einer oder mehreren Wellenlängen. Da Viren sowohl aus Proteinen, als auch aus Nukleotiden bestehen, kann über die Detektion bei 256 - 260 nm (Maximum der Nukleotidabsorption) und 210, 280 nm (Proteinabsorption) virusspezifisch detektiert werden. Bei sehr großen Viren verhindert die Lichtstreuung die direkte Identifizierung über die UV-Absorption (vgl. Bsp. 2.3). Die Lichtstreuung kann aber bei bekannten Viren zur Identifizierung und Quantifizierung herangezogen werden. Der Trennpuffer in der Kapillare ist an das Trennproblem und die Stabilität des Virus anzupassen (pH-Wert, Ionenstärke, Detergenzien, Additive). Apparative Voraussetzung ist ein beliebiges CE-Gerät mit einem optischen Detektor. Die Probenaufgabe in die Kapillare kann elektrokinetisch, hydrodynamisch, über Vakuuminjektion oder Druckaufgabe erfolgen. Es bestehen keine Einschränkungen bezüglich der Kapillare (Quarzkapillare, beschichtet oder unbeschichtet, Teflon oder jedes andere organische oder anorganische Material). Auch der Kapillardurchmesser ist für die Trennung unerheblich und nur durch die Elektrophoresebedingungen limitiert (Stromtransport, Detektierbarkeit, Wärmeabfuhr).

### Ausführungsbeispiele

### 1. Trennung und direkte Identifizierung von MKS-Viren

Maul- und Klauenseuche (MKS)-Viren gehören zur Gruppe der Picornaviren und haben eine ikosaedrische Oberfächenstruktur die aus vier Virusproteinen aufgebaut ist. Der Durchmesser beträgt ca. 25 nm. Im Innern befindet sich eine einsträngige RNA mit einer Länge von ca. 5000 Basen.

Die Vermehrung und Gewinnung der Viren ist in Tabelle 1 zusammenfassend dargestellt.

**Tabelle 1**

| Virusgewinnung MKS | |
|---|---|
| Virusvermehrung | BHK-21 Suspensionszellen in Fermenterkulturen bis 1 500 l, Ernte der Viren nach Zellysis |
| Vorreinigung | Vorklärung der Rohvirussuspension durch niedrigtourige Durchflußzentrifugation |
| Inaktivierung | Ethylenimin (in situ aus Bromethylenamin und NaOH) |
| Konzentratherstellung | PEG-Fällung und Resuspension in 1/100 des Ursprungsvolumen |
| Aufreinigung der Referenzproben | CsCl-Dichtegradientenzentrifugation zur Gewinnung von 146S-Partikeln (vollständige, inaktive Viren) |

### Beispiel 1.1: Identifizierung eines inaktivierten MKS-Virus.

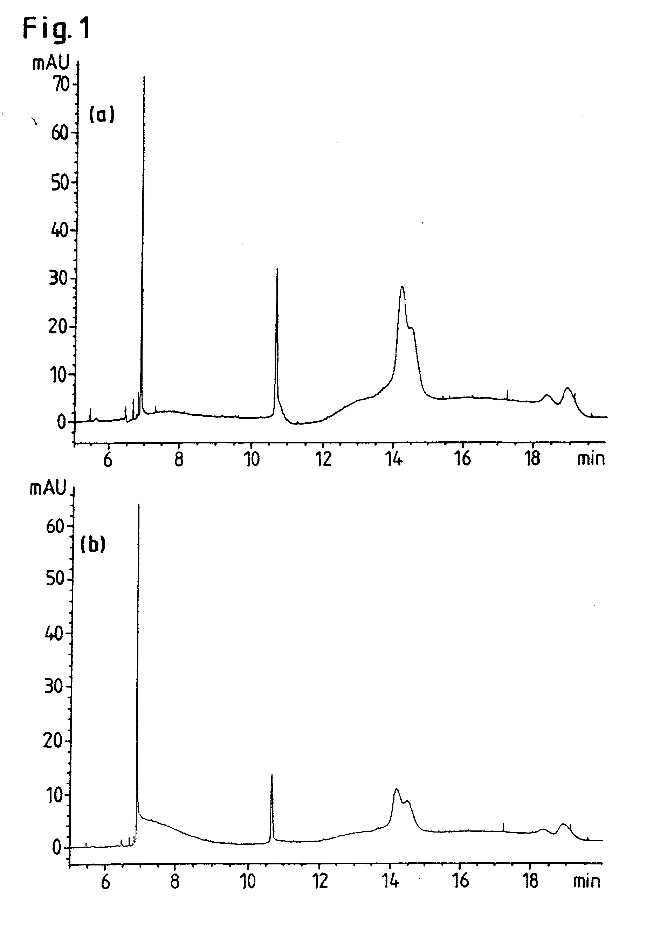

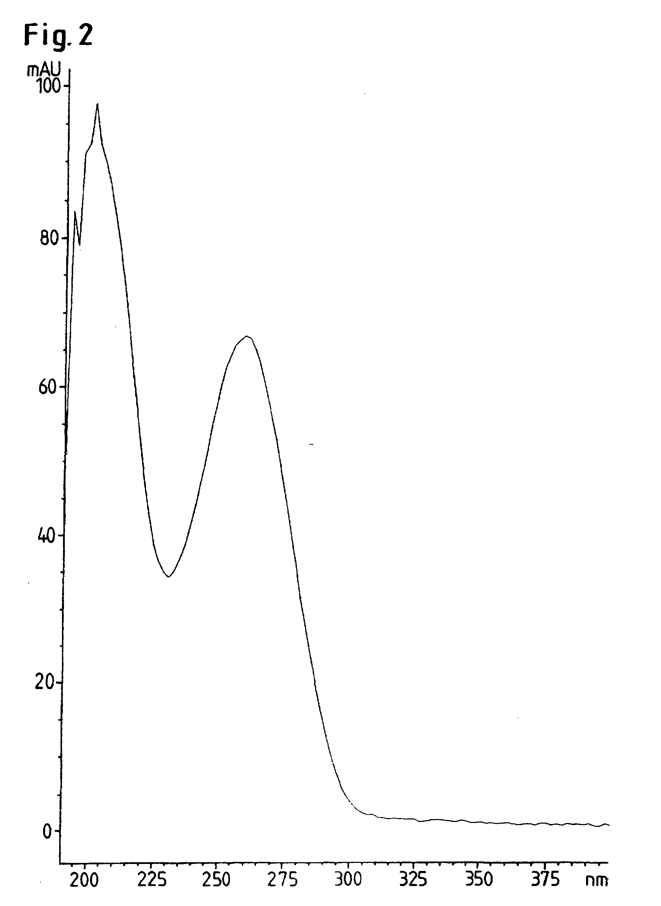

Eine Referenzprobe (vergl. Tab. 1) (100 µg/ml) wurde kapillarelektrophoretisch in einem Kapillarelektrophorese-Gerät der Firma Hewlett-Packard (Typ ^{3D}HPCE) getrennt. Die Meßparameter sind in Tabelle 2 zusammengefaßt. Das zugehörige Elektropherogramm ist in Abbildung 1 dargestellt. Das scharfe Signal bei 6.9 min wächst beim Übergang von 210 auf 257 nm deutlich heraus. Mit Hilfe eines Dioden-Array-Detektors (DAD) wurde das UV-Spektrum dieses Signals aus Abbildung 1 direkt aufgenommen. Das Maximum bei 256 nm ist charakteristisch für Nukleotide, während das intensive kurzwellige Maximum und der allmähliche Abfall auf 300 nm nur über zusätzliche Proteinanteile erklärt werden kann (Abb. 2). Das in der Kapillare gemessene UV-Spektrum war identisch mit dem UV-Spektrum des isolierten Virus und konnte somit für die direkte Identifizierung des Virussignals benützt werden. Keine der Nebenkomponenten in Abbildung 1 zeigte ein analoges UV-Spektum und stellte daher auch keine intakten Viruspartikel dar.

**Tabelle 2**

| Meßparameter zur direkten Identifizierung von MKS-Referenzproben mittels CE | |
|---|---|
| Kapillare | Quarzkapillare 75 µm Innendurchmesser, 64,5 cm Länge, 56 cm zum Detektor |
| Spannung | 20 kV |
| Puffer | Tris (25 mmolar), Glycin (192 mmolar), pH 8,5 |
| Temperatur | 25°C |
| Detektion | DAD 190-600 nm, λ₁ 210 ± 8 nm, λ₂ 257 ± 8 nm |
| Injektion | Druck (20 sec x 5 x 10³ Pa) |
| Spülen der Kapillare vor der Injektion | 1. NaOH (0,1 N, 1 min, 5 x 10⁴ Pa) |
| | 2. Puffer (3 min, 5 x 10⁴ Pa) |

### Beispiel 1.2: Trennung und Identifizierung des inaktivierten MKS-Virus aus, einer komplexen Probenmatrix.

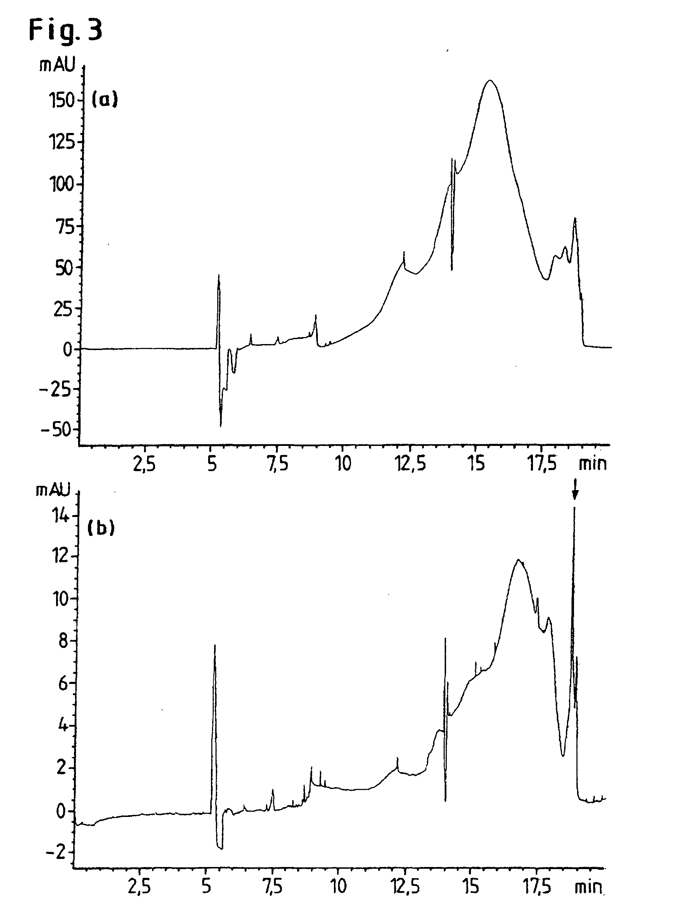

Das inaktivierte Virus konnte im Konzentrat (Vergl. Tab. 1) direkt nachgewiesen werden (Abb. 3, Meßbedingungen in Tab. 3). Bei 210 nm zeigte sich erwartungsgemäß ein komplexes Elektropherogramm. Der biologische Untergrund wurde beim Übergang von 210 auf 257 nm deutlich geringer und das scharf herauswachsende Signal weist auf einen deutlichen Nukleotidgehalt hin. Das Virussignal (Pfeil) konnte über das UV-Spektrum und durch Coinjektion mit der entsprechenden Referenzprobe nachgewiesen werden (ohne Abb.). Eine einfachere und direkte Identifizierung wird in Beispiel 2.2 gezeigt.

**Tabelle 3**

| Meßparameter zur direkten UV-spektroskopischen Identifizierung von MKS-Viren aus Rohkonzentraten | |
|---|---|
| Kapillare | Quarzkapillare 50 µm Innendurchmesser, 64,5 cm Länge, 56 cm zum Detektor |
| Spannung | 20 kV |
| Puffer | Tris (25 mmolar), Glycin (192 mmolar), Natriumdodecylsulfat (50 mmolar), pH 8,5 |
| Temperatur | 25°C |
| Detektion | DAD 190-600 nm, λ₁ 210 ± 8 nm, λ₂ 257 ± 8 nm |
| Injektion | Druck (20 sec x 5 x 10³ Pa) |
| Spülen der Kappilare vor der Injektion | 1. NaOH (0,1 N, 1 min, 5 x 10⁴ Pa) |
| | 2. Puffer (3 min, 5 x 10⁴ Pa) |

### Beispiel 1.3: Trennung und Identifizierung von aktivem MKS-Virus.

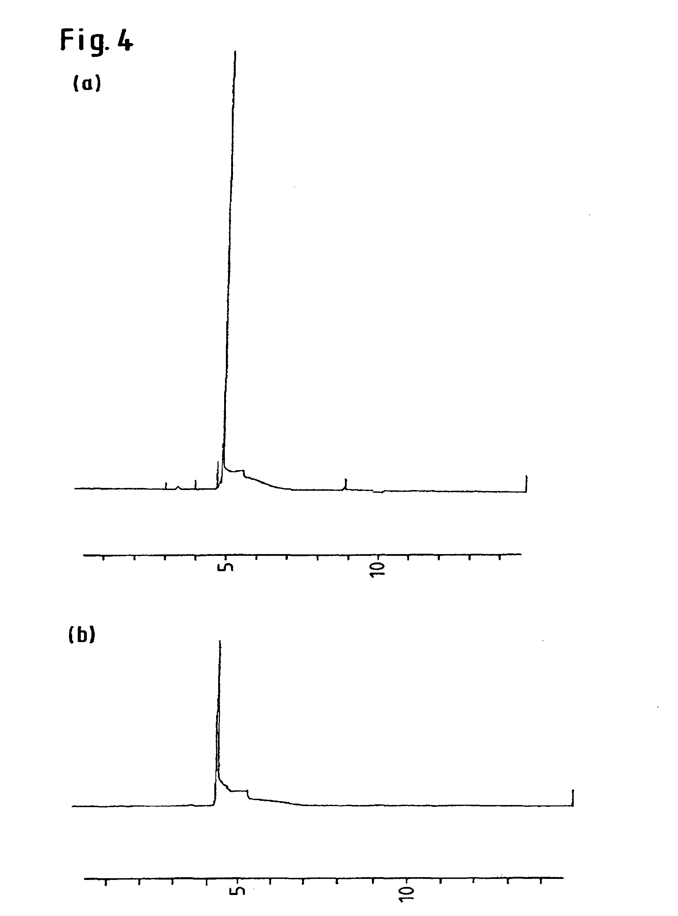

Vom Virusmaterial wurden vor und nach der Inaktivierung Referenzproben gereinigt (vergl. Tab. 1). Die Aktivität des Virus wurde durch Titration in BHK-Zellkulturen überprüft (Titer > 10⁷ KID₅₀). Beide Proben (aktiv und inaktiv) wurden auf die gleiche Menge an 146S-Parikeln eingestellt und mittels eines Kapillarelektrophorese-Geräts der Firma Perkin Elmer (Typ ABI 270 A-HT) getrennt (Meßbedingungen in Tab. 4). Die Detektion bei 257 nm zeigte für beide Präparationen vergleichbare Elektropherogramme (Abb. 4). Das aktive und inaktive Virus konnte mittels CE nicht getrennt werden. Da das Inaktivierungsreagenz auch bevorzugt mit der RNA im Innern des MKS-Virus reagiert, war eine Veränderung des für die CE-Trennung ausschlaggebenden Oberfächenladungszustands auch nicht zu erwarten.

**Tabelle 4**

| Meßparameter zur CE-Trennung von Referenzproben aktiver MKS-Viren | |
|---|---|
| Kapillare | Quarzkapillare 50 µm Innendurchmesser, 72 cm Länge, 51 cm zum Detektor |
| Spannung | 20 kV |
| Puffer | Tris (25 mmolar), Glycin (192 mmolar), pH 8,5 |
| Temperatur | 35°C |
| Detektion | UV 257 nm |
| Injektion | Vakuum(5 sec x 17 x 10³ Pa) |
| Spülen der Kappilarevor der Injektion | 1. NaOH (0,1 N, 1 min, 68 x 10³ Pa) |
| | 2. Puffer (3 min, 68 x 10³ Pa) |

Die Beispiele 1.1 - 1.3 zeigen eindeutig die Eignung dieser neuen Analysenmethode zur direkten Identifizierung von aktiven Viren, wie auch von inaktivierten viralen Partikeln (inaktivierte Proben). Auch komplexe Probenmatrices, wie sie beispielsweise im Konzentrat vorkommen, behindern die Trennung nicht. Hier kommt die hohe Trennleistung der Kapillarelektrophorese und die gute Matrixverträglichkeit des festphasentrennmaterialfreien Analysenverfahrens sehr vorteilhaft zum Tragen. Mit diesen Beispielen konnte das generell neue Verfahren erfolgreich demonstriert und eingesetzt werden. Viren können mit diesem neuartigen Analysenverfahren direkt detektiert und identifiziert werden. Die Peakfläche des Virussignals korreliert mit der Virusmenge und ist daher für die direkte Quantifizierung der Viruskonzentration geeignet. Am Beispiel des MKS-Virus konnte die UV-Absorption zur direkten Identifizierung des Virussignals herangezogen werden. Bei sehr großen Viren verhindert die Lichtstreuung die direkte UV-spektroskopische Identifizierung des Virus. Auch ist das Detektionslimit für UV-absorbierende Viren im Mikrogramm pro Milliliterbereich (>10⁶ Teilchen/ml) und damit für viele Zwecke ungenügend. Im nächsten Kapitel sollen Verfahren aufgezeigt werden, die auch für diese Viren ein spezifisches Detektionssystem bereitstellen.

### 2. Identifizierung von Viren mit einem RNA/DNA spezifischen Detektionssystem auf der Basis von DNA/RNA bindenden Farbstoffen

Nachfolgend wird beschrieben wie DNA/RNA bindende Farbstoffe zur direkten Anfärbung von Viren eingesetzt werden können und in Erweiterung des unter Punkt 1 erläuterten Trennverfahrens für Viren und virale Partikel dadurch zum einen die Zuordung des Signals, speziell in komplexen Systemen beträchtlich erleichtert wird, wenn nicht gar erst möglich gemacht wird und zum anderen durch die Fluoreszenzdetektion eine weitere Spezifizierung und vor allem eine drastische Empfindlichkeitssteigerung erreicht wird.

Durch die CE-Trennung können die so angefärbten Viren von anderen DNA/RNA enthaltenden Komponenten abgetrennt werden. So kann das markierte Virus direkt identifiziert werden. Über die Selektivität der Farbstoffe für DNA oder RNA, bzw. Doppelsirang oder Einzelstrang kann eine zusätzliche Virusspezifizierung erreicht werden. Über die unterschiedliche Membrangängigkeit der Farbstoffe können auch umhüllte und unbehüllte Viren differenziert werden.

Die Fluoreszenzdetektion führt durch den mehrfachen Einbau der Farbstoffe (alle 10 bis 20 Basenreste) zu einer extremen Steigerung der Empfindlichkeit. Es werden damit statistisch bis zu 15 000 Farbstoffmoleküle eingebaut, von denen jedes zum Fluoreszenzsignal beiträgt. Rein rechnerisch ist damit für bestimmte Viren die Nachweisgrenze mit Fluoreszenzdetektion unterhalb der eines Partikels.

Es werden daher erstmals angefärbte Viren mit der CE getrennt, anhand der charakteristischen UV-Absorption identifiziert und über die Fluoreszenz hochsensitiv detektiert. Basierend auf den Arbeiten aus den Beispielen 1.1 - 1.3 wurden MKS-Viren mit DNA/RNA bindenden Farbstoffen versetzt und mittels CE und DAD-Detektion untersucht. Die langwelligen Absorptionsmaxima der angefärbten Viren entsprechen in erster Näherung den Absorptionsmaxima der angefärbten, freien RNA oder DNA.

### Allgemeine Beschreibung des Anfärbeverfahrens

Die zu analysierende Probe wird:
a) mit dem DNA/RNA bindenden Farbstoff inkubiert und anschließend entsprechend dem unter Punkt 1 beschriebenen Verfahren getrennt und analysiert oder
b) dem Trennpuffer wird der DNA/RNA bindende Farbstoff zugesetzt und das Virus während der Trennung in der Kapillare angefärbt. Das Virus mit DNA/RNA gebundenem Farbstoff kann entweder durch die spezifische UV/VIS-Absorption oder durch die spezifische Extinktion und Emission der Fluoreszenz detektiert werden.

### Ausführungsbeispiele

Von den zahlreichen, erfolgreich eingesetzten Anfärbedetektionsverfahren mit unterschiedlichen Viren und unterschiedlichen Farbstoffen sollen exemplarisch 2 unterschiedliche Systeme dargestellt werden.

Zunächst wird die Trennung, Identifizierung und hochempfindliche Detektion von angefärbten MKS-Viren beschrieben.

Durch Anfärben mit den DNA/RNA bindenden Farbstoffen verschiebt sich das kurzwellige Absorptionsspektrum und zusätzlich kommt eine Absorption im sichtbaren Bereich hinzu. Zuerst soll daher die UV-Absorption zum Nachweis der erfolgreichen Trennung der angefärbten Viren dienen und außerdem über das charakteristische Absortionsspektrum das Virus unabhängig identifiziert werden.

Da MKS-Viren einsträngige RNA enthalten und die Anfärbereagenzien für doppelsträngige DNA entwickelt wurden, kommt ein weiteres erschwerendes Kriterium hinzu. Durch Voruntersuchungen konnte jedoch gezeigt werden, daß freie einsträngige DNA und RNA ebenfalls, wenn auch in geringerem Maße, angefärbt und mittels CE detektiert werden konnte.

### Beispiel 2.1: Trennung und Identifizierung von angefärbten MKS-Viren mit UV-Detektion.

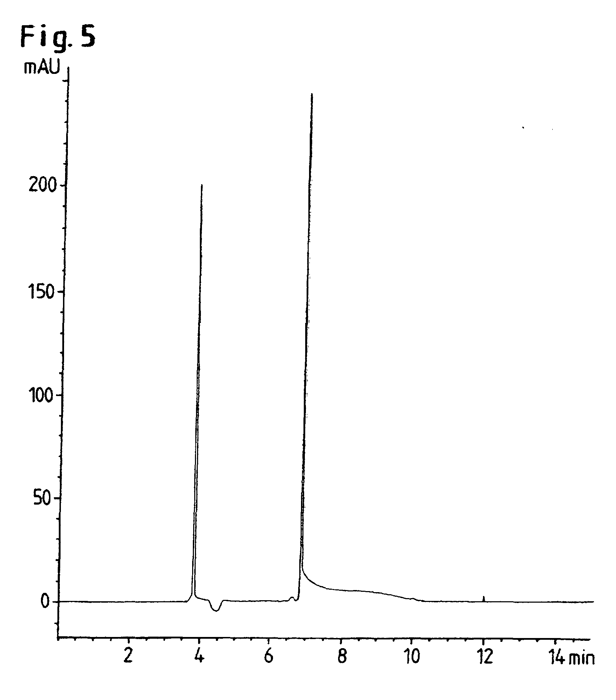

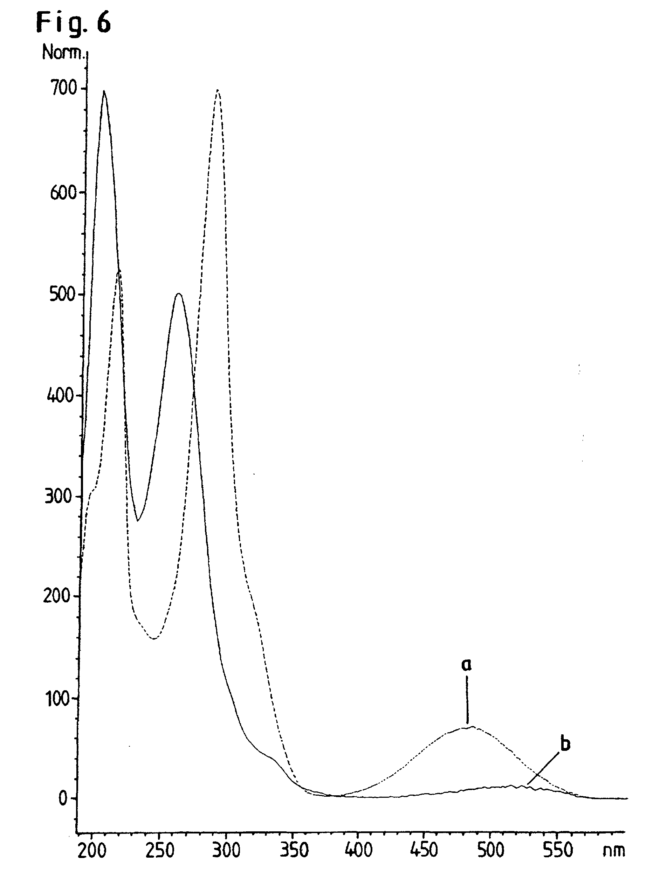

Die Referenzprobe von MKS aus Beispiel 1.1 (300 µg/ml) wurde mit 10 µg/ml Ethidiumbromid (s. z.B. R.P. Haugland, Handbook of fluorescent probes and research chemicals, Molecular Probes, Inc. 1992) für 30 min bei Raumtemperatur inkubiert und dann mit der CE (Bedingungen gem. Tab. 2) untersucht. Es waren zwei Signale detektierbar (Abb. 5). Die UV/VIS-Spektren der Signale in der CE sind in Abbildung 6 dargestellt. Das Signal bei 3.8 min stellte noch überschüssiges Ethidiumbromid aus der Inkubation dar (auch durch Coinjektion nachgewiesen). Der Peak bei 6.9 min zeigte Absorptionsmaxima bei 200, 257 (intensiv) und 510 nm (schwach). Die langwellige Verschiebung des sichtbaren Absorptionsmaximums von freiem Ethidiumbromid (495 nach 510 nm) ist charakteristisch für die Interkalation mit Nukleobasen. Durch den Einbau von Ethidiumbromid wurde die Lage des Virussignals im Elektropherogramm praktisch nicht verschoben (vergl. Abb. 1 und 5).

Am Beispiel von Ethidiumbromid konnte die Trennung und Identifizierung angefärbter Viren erfolgreich demonstriert werden.

### Beispiel 2.2: Fluoreszenzdetektion von angefärbtem MKS-Virus.

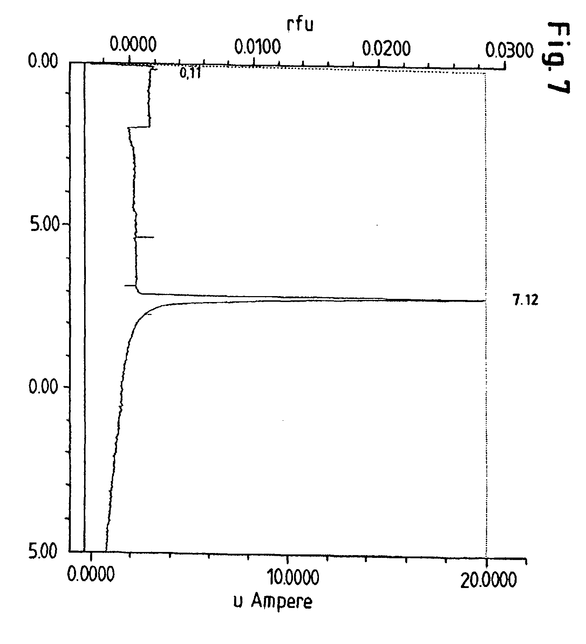

Die MKS-Konzentratprobe aus Beispiel 1.2 wurde nach 1:15 Verdünnung mit dem Trennpuffer (Tab. 5) mit 1,1'-(4,4,7,7-Tetramethyl-4,7diazaundecamethylen)-bis-4-[3-methyl-2,3-dihydromethyl-(benzo-1,3-oxazol)-2methyliden)-chinolinium Tertaiodid (YOYO) (1 µmolar) für 30 min bei Raumtemperatur inkubiert und dann mit der CE (Meßbedingungen in Tab. 5) gemessen. Es war nur noch ein einziges Signal im Konzentrat zu sehen (Abb. 7). Die Eigenfluoreszenz von YOYO war so gering, daß freies YOYO mit der Fluoreszenzdetektion nicht mehr detektiert werden konnte. Die restlichen Signale aus dem Konzentrat wurden durch diese nukleotidspezifische Detektion unsichtbar (vergleiche UV-Detektion der gleichen Probe in Abbildung 3). Die starke Verschiebung in der Migrationszeit ergab sich aus unterschiedlichen Kapillarlängen und Feldstärken (vergl. Tab. 3 und Tab. 5).

**Tabelle 5**

| Meßparameter zur CE-Trennung mit Fluoreszenzdetektion von MKS-Referenz-proben mittels CE | |
|---|---|
| Kapillare | Quarzkapillare 50 µm Innendurchmesser, 47 cm Länge, 40 cm zum Detektor |
| Spannung | 20 kV |
| Puffer | Tris (25 mmolar), Glycin (192 mmolar), Natriumdodecyl-sulfat (50 mmolar), pH 8,5 |
| Temperatur | 25°C |
| Detektion | LIF (Argon) EX 488, EM 520 nm, Gain 100 |
| Injektion | Druck (10 sec x 10³ Pa) |
| Spülen der Kappilare vor der Injektion | 1. NaOH (0,1 N, 1 min, 5 x 10⁴ Pa) |
| | 2. Puffer (3 min, 10⁴ Pa) |

### Beispiel 2.3: Trennung und Identifizierung von Orf-Viren mit Fluoreszenzdetektion.

Das Orf-Virus gehört zur Gruppe der Parapoxviren. Das Virus besteht aus einer doppelsträngigen DNA mit ca. 140 000 Basenpaaren, die zusammen mit einer Vielzahl an Enzymen in einer Proteinhülle (Core) und einer weiteren Lipoproteinhülle eingeschlossen ist. Es handelt sich daher um ein sogenanntes umhülltes Virus. Das Viruspartikel hat eine Backsteinstruktur mit einer Größe von ca. 400 x 200 x 200 nm und gehört daher zu den größten Viren überhaupt. Aufgrund der zu MKS sehr unterschiedlichen Oberflächenstruktur und Größe soll mit diesem Beispiel die Anwendungbreite des neuen Verfahrens demonstriert werden. Die Virusgewinnung wurde wie folgt durchgeführt (Tab. 6):

**Tabelle 6**

| Virusgewinnung ORF | |
|---|---|
| Virusvermehrung | BK-Klon 3 A Monolayerzellen in Wannenstapeln; 150 l Virusernte nach Zellysis |
| Vorreinigung | VorKlärung der Rohvirussuspension durch niedrigtourige Durchflußzentrifugation |
| Inaktivierung | β-Propiolacton |
| 2. Reinigungsschritt | niedrigtourige Durchflußzentrifugation |
| Aufreinigung der Referenzproben | Dichtegradientenzentrifugation in Natriumtartrat zur Gewinnung der Virusbande |

Eine direkte UV-spektroskopische Identifizierung scheidet wegen der Größe des Virus aus, da bei dieser Größe im UV bereits Lichtstreuung auftritt. Außerdem liegt die Orf-Probe auch nach dem zweiten Reinigungschritt (Tab. 6) in einer zu geringen Konzentration für die UV-Detektion vor. Das Virus kann daher nur über eine Fluoreszenzdetektion erfaßt werden.

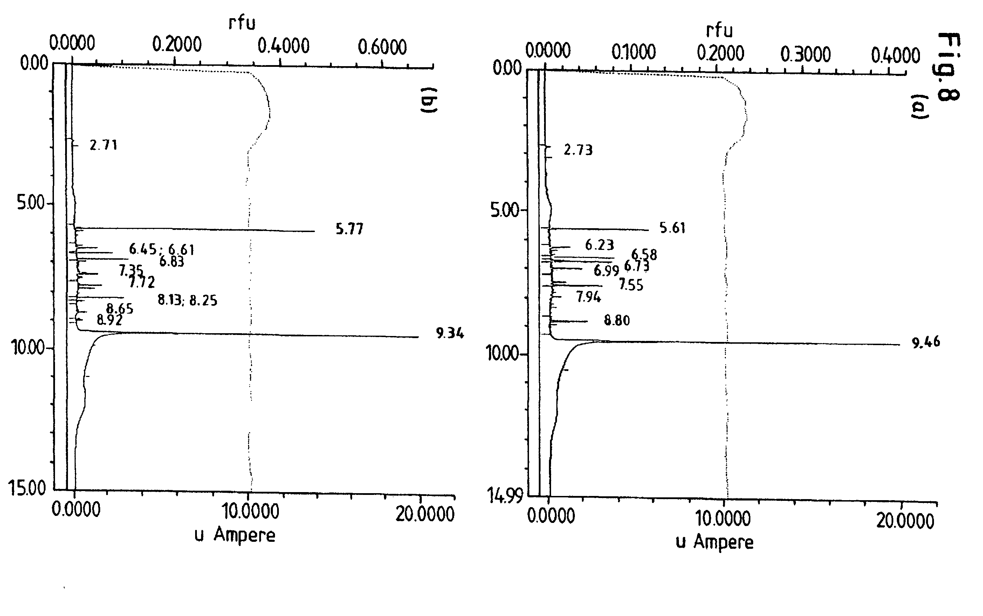

Baypamun® (kommerziell erhältlicher Immunmodulator der Fa. Bayer AG aus inaktivierten Orf-Viren) wurde mit YOYO (1 µmolar) für 30 min bei Raumtemperatur inkubiert und dann mit der CE getrennt (Meßbedingungen wie in Tab. 5, aber Trennpuffer war Tris / Borat, 100 mmolar pH 8.5). Bei 2.73 min war das überschüssige YOYO als extrem kleiner Peak zu sehen (Abb. 8.a). Das Orf Virus konnte bei 9.46 min als einzelnes Signal abgetrennt und detektiert werden. Die zusätzlich auftretenden Spikes wurden wohl durch die Probenmatrix hervorgerufen, da in diesem Bereich des Elektropherogramms mit UV eine breite Absorption gemessen werden konnte. Die Spikes traten bei dieser Messung immer auf. Die Lage der Spikes war aber nicht reproduzierbar.

Mit Hilfe der Fluoreszenzdetektion konnte also ein extrem großes umhülltes Virus direkt nachgewiesen werden. Eine zusätzliche Identifizierung des Signals wird in Beispiel 3.2 beschrieben.

### Beispiel 2.4: Trennung und Identifizierung der Core des Orf-Virus

Wie bereits in der Einleitung zu Beispiel 2c ausgeführt, verhindern die Größe und die Konzentration von Orf die direkte UV-Detektion. Da es sich außerdem um ein umhülltes Virus handelt, sollte versucht werden den inneren Proteinmantel inklusive der eingeschlossenen DNA (Core) zu untersuchen.

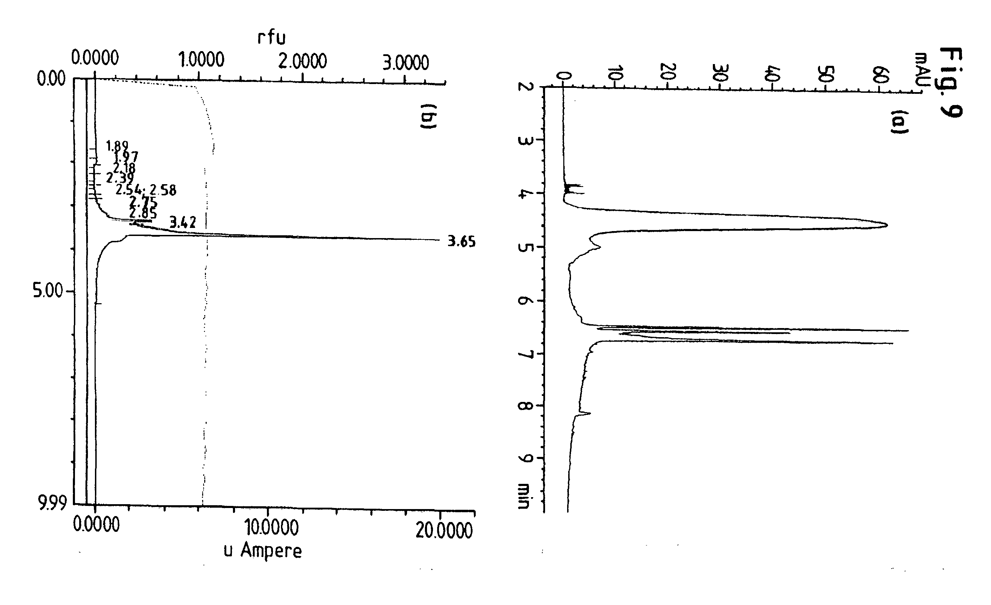

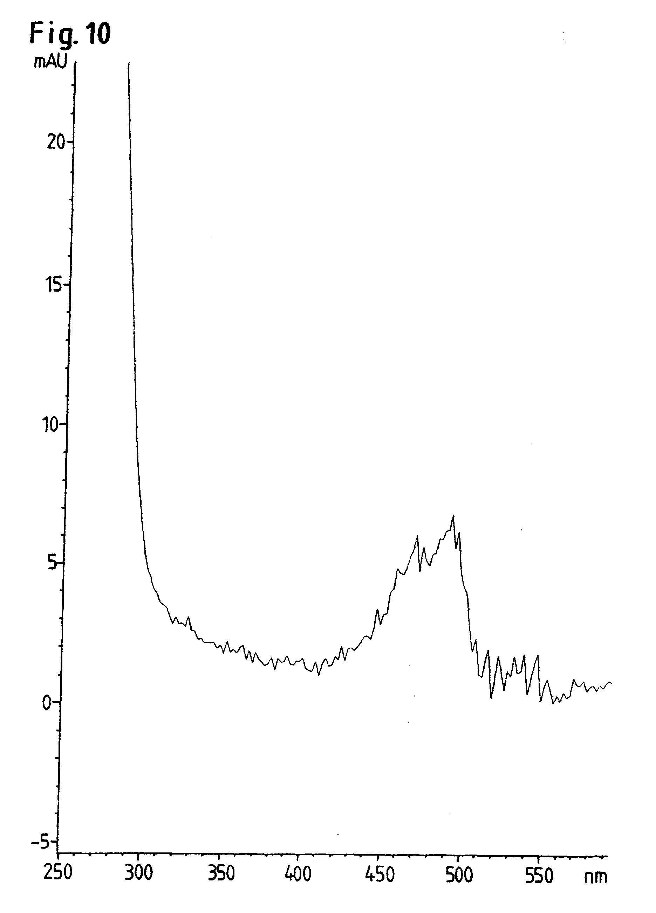

Baypamun wurde nach modifizierter Vorschrift [9] durch Schütteln mit NP-40 (1 %; 1 h, 37 °C) von seiner Lipoproteinmembran befreit und die Core durch Dichtegradientenzentrifugation (Succrose 36 %, PBS 150 mmolar, 225,000 g, 1 h) pelletiert. Die so aufkonzentrierte Probe wurde mit YOYO (Konzentration 1 µmolar) für 30 min bei Raumtemperatur inkubiert und dann mit einer CE mit DAD (Meßbedingungen in Tab. 2) gemessen. Das Elektropherogramm bei 257 nm (Abb. 9.a) zeigt eine breite Bande bei 4.5 min, die als überschüssiges YOYO identifiziert werden konnte. Von den drei intensiven Signalen zwischen 6.4 und 6.8 min zeigte nur das letzte Signal das für interkalierendes YOYO typische Absorptionsmaximum bei 495 nm (Abb. 10). Die identische Probe wurde anschließend auch noch mit der LIF-Detektion untersucht. Die Meßbedingungen entsprachen denen aus Tabelle 5, nur auf Natriumdodecylsulfat im Trennpuffer wurde verzichtet (gleicher Puffer wie bei der UV-Detektion). Die absolute Migrationszeit war durch den Wechsel der Kapillare und der Feldstärke gegenüber der UV-Messung deutlich verschoben (Abb. 9). Das überschüssige YOYO konnte als winziger Peak bei 1.9 min identifiziert werden. Von den drei UV-Signalen konnte nur das angefärbte Produkt (die Core) detektiert werden. Zusätzlich konnte durch die Fluoreszenzdetektion eine Steigerung in der Empfindlichkeit um den Faktor 10⁴ gegenüber der UV-Detektion der nicht angefärbten Core erreicht werden.

Mit diesen Experimenten konnte bewiesen werden, daß nicht nur komplette Viren (umhüllt oder unbehüllt) direkt identifiziert werden können, sondern auch veränderte Viruspartikel, wie sie aus umhüllten Viren durch Entfernen der Lipoproteinmembran hergestellt werden können.

Im nächsten Kapitel wird beschrieben, wie durch eine zusätzliche Spezifizierung der Virussignale eine weiterere Verbesserung erreicht wird.

### 3. Zusätzliche Spezifizierung der Viruspartikel während (bei) der kapillarelektrophoretischen Trennung durch Antikörperwechselwirkungen

Es wurde untersucht, wie mit spezifischen Antikörpern (MAb) bekannte Viren eindeutig differenziert werden. Damit steht die gesamte etablierte immunologische Virusdifferenzierung in direkter Kombination mit der Trennung der Viren in der CE zur Verfügung.

### Allgemeine Beschreibung des Verfahrens

Die zu analysierende Probe wird:
a) mit dem Antikörper (MAb, Antiseren, Antikörpermischung, markierte Antikörper) inkubiert und anschließend entsprechend den vorbeschriebenen Verfahren getrennt und analysiert oder
b) dem Trennpuffer wird der Antikörper (MAb, Antiseren, Antikörpermischung, markierte Antikörper) zugesetzt und das Virus entsprechend einer Affinitäts-Kapillarelektrophorese unter Berücksichtigung der vorbeschriebenen Verfahren getrennt und analysiert.

Die spezifische Wechselwirkung des (der) Antikörper mit dem Virus kann entweder durch eine Migrationszeitverschiebung oder durch eine Abnahme der Signalintensität des Virussignals detektiert werden.

### Ausführungsbeispiele

In den nachfolgenden Beispielen wurde das zu untersuchende Virus mit spezifischen Antikörpern inkubiert und anschließend mit der CE getrennt. Die Stabilität der Antikörper-Antigen-Bindung ist groß genug, um in nicht denaturierenden Lösungsmitteln die Trennung als intakter Komplex zu überstehen. Gemessen werden kann somit der Unterschied in der Migrationszeit zwischen dem freien Virus und dem Virus-Antikörper-Komplex aus unterschiedlichen Trennungsläufen.

### Beispiel 3.1: Spezifizierung des Orf-Virussignals durch Migrationszeitverschiebung mit monoklonalem Antikörper.

Die Orf-Probe aus Beispiel 2.3 wurde nach der Inkubation mit dem Farbstoff noch mit einem Anti-Orf Antikörper (MAb) versetzt (2 µg/ml) und weitere 30 min bei Raumtemperatur inkubiert. Die Meßbedingungen waren mit denen in Beispiel 2.3 identisch. Das Elektropherogramm ist in Abbildung 8.b dargestellt. Das Elektropherogramm unterscheidet sich von dem des freien Virus lediglich durch die Lage des Virussignals (Abb. 8). Das Fluoreszenzsignal des Orf Virus verschob sich durch die Bindung des MAb von 9.46 min reproduzierbar auf 9.34 min. Eine stärkere Verschiebung war wegen der Größe des Virus nicht zu erwarten. Da dieses Virus zu den größten Viren überhaupt gehört, konnte mit diesem Experiment die Anwendungsbreite dieses Verfahrens demonstriert werden. Die Verschiebung des Signals beweist, daß das beobachtete Fluoreszenzsignal tatsächlich viralen Ursprungs ist und bei hinreichender Spezifität des MAb auch dem Orf-Virus zugeordnet werden kann. Daß mit kleineren Viren eine deutlich stärkere Verschiebung erreicht werden kann, soll im nächsten Beispiel gezeigt werden.

### Beispiel 3.2: Spezifizierung der MKS-Virussignate durch Migrationszeitverschiebung mit monoklonalen Antikörpern.

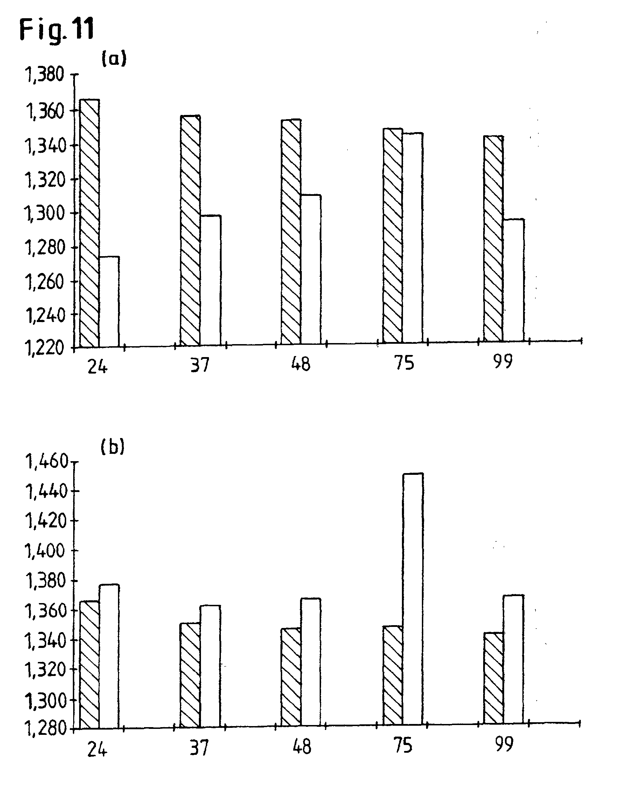

Die Referenzprobe aus Beispiel 1.1 (100 µg/ml) entspricht dem aus der Literatur bekannten Virusstamm O₁ₖ (O₁-Kaufbeuren) und wurde mit unterschiedlichen Anti-MKS-O₁-MAb (1-10 µg/ml) und Anthrachinon-1-sulfonsäure als internem Standard (2 µg/ml) versetzt und 30 min bei Raumtemperatur inkubiert und mit den Bedingungen aus Tabelle 2 gemessen. Die relativen Migrationszeiten (tₘᵣ) der inkubierten Proben wurden mit den tₘᵣ des reinen Virus bei gleicher Probenvorbereitung und identischen Meßbedingungen verglichen (Abb. 11.a). Die dunklen Balken zeigen die tₘᵣ des reinen Virus, die hellen Balken die der antikörperinkubierten Viren. Die unterschiedlichen MAb sind in der x-Achse aufgetragen und wurden in der Reihenfolge von links nach rechts gemessen. An den dunklen Balken ist ein geringfügiger, anfänglicher Abfall der tₘᵣ zu erkennen, der auf eine noch nicht vollständig äquilibrierte Kapillare zurückzuführen ist. Für Antikörper 24,37,48 und 99 konnte eine zum Teil deutliche Verschiebung der tₘᵣ beobachtet werden. Die untersuchte Referenzprobe konnte damit serologisch dem Stamm O₁ zugeordnet werden. Das Ausmaß der Verschiebung ist kein direktes Maß für die Affinität des MAb. Die Verschiebung wird durch das Migrationsverhalten und die Anzahl der gebundenen Antikörper bestimmt. Die positiv auffallenden MAb zeigten alle eine Verschiebung der tₘᵣ zwischen 0.05 und 0.13. Antikörper 75 führte zu keiner signifikanten Verschiebung der tₘᵣ, eine Erkennung des Virus durch diesen Antikörper war somit nicht nachweisbar. Entweder waren die Migrationszeiten von MAb 75 und MKS-O₁ₖ zu ähnlich oder MAb 75 erkennt diesen Virusstamm nicht.

Um die Spezifität der MAb-Bindung an MKS-O₁ₖ zu verifizieren wurden die gleichen Antikörper unter den identischen Bedingungen mit MKS-O₁ₘ inkubiert und vergleichend gemessen. (O₁ₘ (O1-Manisa) ist ein anderer, aus der Literatur bekannter Virusstamm). Auch hier ist an den dunklen Balken der anfängliche Abfall der tₘᵣ erkennbar. Nach Inkubation mit den MAb (helle Balken) zeigt nur MAb 75 eine signifikante Verschiebung um 0.10. Dieser MAb erkennt demnach eindeutig MKS-O₁ₘ. Da MKS-O₁ₘ und MKS-O₁ₖ bei sehr ähnlichen tₘᵣ detektiert wurden, scheidet eine Nichterkennung von MKS-O₁ₖ aufgrund ähnlicher Migrationszeiten von Virus und Virus-MAb-Komplex aus. MAb 75 konnte daher als Anti-MKS-O₁ₘ bestätigt werden, während alle anderen MAb nur MKS-O₁ₖ erkennen. Die restlichen MAb verschieben das MKS-O₁ₘ um maximal 0.02, was im Vergleich zu Abbildung 11.a als unspezifisch angesehen werden muß.

Aus den vorbeschriebenen Messungen geht eindeutig hervor, daß mit dem neu entwickelten Verfahren Viren und Viruspartikel (inaktivierte Viren, Core) direkt analysiert und detektiert werden können. Auch komplexe Probenmatrices (Rohkonzentrat von MKS, Baypamun) beeinträchtigen die Analyse nicht. Am Beispiel der kleinen Picomaviren (MKS), dem großen umhüllten Orf-Virus und dessen Core konnte die Anwendungsbreite dieses Verfahren auf unterschiedlichste Viren demonstriert werden. Durch nukleotidspezifische Anfärbereagenzien konnten Viren sowohl UV- als auch fluoreszenzspezifisch detektiert werden. Dabei konnten drastische Empfindlichkeitssteigerungen beobachtet werden. Über die Komplexierung mit Antikörpern können die Viren oder viralen Partikel spezifisch detektiert werden. Die Antikörpererkennung konnte auch in Gegenwart der Anfärbereagenzien dokumentiert werden. Damit steht ein komplettes Analysenrepertoir zur generellen Erfassung viraler Komponenten in komplexen Matrices zur Verfügung. Dieses Verfahren bietet daher ein erhebliches Anwendungspotential, sowohl für die Prozeßkontrolle, wie auch für die Virusdiagnostik.

### Literatur

- [1]: Engelhardt, H., Beck, W., Kohr, J. und Schmitt, T. Angew. Chem. **1993,** 105, 659-680
- [2]: Kuhr, W.G. und Monnig, C.A. Anal. Chem. **1992**, 64, 389R-407R
- [3]: W.M. Hurni und W.J. Miller, J. Chromato. **1991**, 559, 337-343
- [4]: R. Bartzatt, J. Histotechnol. **1987**, 10, 95-96
- [5]: Haugland, RP., Handbook of fluorescent probes and research chemicals (Larison, K.D., Hrsg.) Molecular Probes, Inc. **1992**
- [6]: Zuh, H., Clark, S.M., Benson, S.C., Pye, H.S., Glazer, A.N. und Mathies, R.A. Anal. Chem. **1994**, 66, 1941-1948
- [7]: Heegard, N.H.H. und Robey, F.A. Anal. Chem. **1992**, 64, 2479-2482
- [8]: Chu, Y.-H., Avila, L.Z., Biebuych, H.A. und Whitesides, G.M.J. Org. Chem. **1993**, 58, 648-652
- [9]: Paoletti, E., Rosemund-Hombeak, und Moss, B.J. Bio. Chem. **1974**, 249, 3273-3280
- [10]: Hjerten, S., Elenbring, K., Kilár, F., Liao, J.-L., Chen, A.j.C., Siebert, C., Zhu, M.-D. Journal of Chromatography **1987,** 403, 47-61

## Patentansprüche

1. Verfahren zum zerstörungsfreien analytischen Nachweis und/oder zur Quantifizierung von Viren oder viralen Partikeln (Analyten) in einer organische oder anorganische Nebenkomponenten, insbes. Proteinanteile und/oder Nucleotide und/oder andere Viren, enthaltenden flüssigen Probenmatrix, wobei
a) die Viren oder die viralen Partikel von den Proteinanteilen und/oder Nucleotiden in der Probenmatrix kapillarelektrophoretisch getrennt werden und dabei das zugehörige Elektropherogramm registriert wird und
b) die den Analyten zugeordneten Fraktionen als Virus-Peaks im Elektropherogramm durch spektroskopische Auswertung an Hand charakteristischer Maxima identifiziert werden,
**dadurch gekennzeichnet, daß**
die Viren oder die viralen Partikel mit einem RNA/DNA-bindenden, spektroskopisch identifizierbaren Fluoreszenzfarbstoff angefärbt werden.

2. Verfahren nach Anspruch 1, **dadurch gekennzeichnet, daß** die Viren oder die viralen Partikel vor der kapillarelektrophoretischen Trennung mit dem RNA/DNA-bindenden Fluoreszenzfarbstoff inkubiert werden.

3. Verfahren nach Anspruch 1, **dadurch gekennzeichnet, daß** dem Trennpuffer der RNA/DNAbindende Fluoreszenzfarbstoff zugesetzt wird und die Viren oder die viralen Partikel während der kapillarelektrophoretischen Trennung in der Kapillare angefärbt werden.

4. Verfahren nach Anspruch 1 - 3, **dadurch gekennzeichnet, daß** der Probenmatrix zur Identifizierung eine Referenzprobe eines bekannten Virus oder eines bekannten viralen Partikels zugesetzt wird.

5. Verfahren nach Anspruch 1 - 4, **dadurch gekennzeichnet, daß** das Virus oder die viralen Partikel mit spezifischen Antikörpern inkubiert werden, die eine Verschiebung der elektrophoretischen Migrationszeit oder eine Änderung des Virus-Peaks hervorrufen.

6. Verfahren nach Anspruch 1 - 5, **dadurch gekennzeichnet, daß** die Herstellung der Viren bei der Vakzineproduktion meßtechnisch überwacht und gesteuert wird.

7. Verfahren nach Anspruch 1 - 5, **dadurch gekennzeichnet, daß** Viren in biologischen Materialien, insbesondere in Körperflüssigkeiten diagnostiziert werden.

## Claims

1. Method for the non-destructive analytical detection or quantification of viruses or viral particles in a liquid sample matrix containing organic or inorganic minor constituents, protein moieties, nucleotides or other viruses, whereby
a) the viruses or the viral particles from the protein moieties and/or nucleotides in the body liquid are separated using a capillary electrophoresis system and recorded in an electropherogram and
b) the fractions are being identified as virus peaks by interpretation of the data in the electropherogram
wherein the virus or the viral particles have been stained with RNA/DNA-binding reagent enabling spectroscopic identification.

2. Method according to claim 1, which comprises the further step of incubation of the virus or the viral particles with RNA/DNA-binding reagents prior to the electrophoretic separation.

3. Method according to claim 1, which comprises the further step of adding the RNA/DNA-binding reagent to the separation buffer thus staining the virus or the viral particles in the capillary during the electrophoretic separation.

4. Method according to claims 1 -3. which comprises the further step of adding a reference sample of a known virus or of a known viral particle to the body liquid for the identification of the fractions.

5. Method according to claims 1-4. which comprises the further step of incubating the virus or the viral particle with specific Antibodies resulting in a shift of electrophoretic migration time or a change in the virus signal.

6. Method according to claims 1-5, which enables the monitoring and control of virus during vaccine production.

7. Method according to claims 1-5, which enables the diagnosis of virus in biological tissue, particularly in body liquid.

## Revendications

1. Procédé de détection analytique non destructif et/ou de quantification de virus ou de particules virales (analytes) dans un composant intermédiaire organique ou anorganique, en particulier une matrice d'échantillon liquide contenant une proportion de protéines et/ou de nucléotides et/ou d'autres virus, dans lequel
a) les virus ou les particules virales dans les proportions de protéines et/ou de nucléotides dans la matrice d'échantillon sont séparés par électrophorèse capillaire, l'électrophérogramme correspondant étant enregistré et
b) les fractions correspondantes des analytes sont identifiées en tant que pic viral dans l'électrophérogramme par une analyse spectroscopique sur la base des valeurs maximales caractérisées,
**caractérisé en ce que** les virus ou les particules virales sont colorées par un fluorochrome identifiable par spectroscopie, se liant à l'ARN/ADN.

2. Procédé selon la revendication 1, **caractérise en ce que** les virus ou les particules virales sont incubés avant la séparation par électrophorèse capillaire avec le fluorochrome se liant à l'ARN/ADN.

3. Procédé selon la revendication 1, **caractérisé en ce que** le fluorochrome se liant à l'ARN/ADN est ajouté au tampon de séparation et que les virus ou les particules virales sont colorées dans le système capillaire pendant la séparation par électrophorèse capillaire.

4. Procédé selon les revendications 1 à 3, **caractérisé en ce qu'**un échantillon de référence d'un virus connu ou d'une particule virale connue est ajouté à la matrice d'échantillon pour identification.

5. Procédé selon les revendications 1 à 4, **caractérisé en ce que** le virus ou les particules virales sont incubées avec des anticorps spécifiques qui entraînent un décalage de la durée de migration électrophorétique ou une modification du pic viral.

6. Procédé selon les revendications 1 à 5, **caractérisé en ce que** la production des virus est surveillée et commandée par le biais d'une technique de mesure lors de la production de vaccin.

7. Procédé selon les revendications 1 à 5, **caractérisé en ce que** les virus sont diagnostiqués dans des matériaux biologiques, en particulier dans des liquides corporels.
